# EUROPEAN PATENT APPLICATION

(11) **EP 4 563 170 A1**
(43) Date of publication of application: **04.06.2025**
(21) Application number: 23213020.3
(22) Date of filing: 29.11.2023
(51) Int. Cl.: A61M 5/00, A61M 5/32, A61M 5/24, A61M 5/34

(54) **NEEDLE DEVICE, MEDICAL DEVICE AND MEDICAL DEVICE SYSTEM**

(71) Applicant: Sensile Medical AG, 4600 Olten (CH)
(72) Inventor: PFRANG, Jürgen, 93183 Kallmünz (DE)
(74) Representative: Bardehle Pagenberg Partnerschaft mbB Patentanwälte Rechtsanwälte

(57) **Abstract**

The present disclosure relates to a needle device for a medical device, in particular for a medical injection device, the needle device comprising: a hub portion (10) defining a longitudinal axis (11), wherein the hub portion (10) comprises a cannula (12) extending from the hub portion (10) substantially along the longitudinal axis (11); a tubular portion (40) arranged at an outer periphery of the hub portion; wherein the needle device is releasably attachable to a housing of a medical device.

## Description

### 1. Technical field

The present disclosure relates to a needle device for a medical device, in particular a medical injection device, a needle device unit, a medical device, a medical device system, and a method for assembling a medical device.

### 2. Prior art

Medical devices, in particular medical injection devices, such as auto-injectors or injection pens, are known in the prior art and popular among users as they provide various benefits and can be used for various applications. Such medical devices are also sometimes referred to as drug delivery devices in particular infusion devices that are used to deliver a medicament to a user, such as a patient.

An auto-injector is a device with a pre-filled syringe, cartridge, or vial containing a medication. It is typically designed to be automated, meaning that a user may not need to manually load the medication or set the dose. Auto-injectors are often designed to be more user-friendly and may have features like automatic needle insertion and medication delivery at the push of a button. An injection pen is device that typically comprises a cartridge or a vial containing the medication and a pen-like device with a needle at the tip. It usually requires manual assembly of the cartridge or vial into the pen and the setting of the desired dose before injection. The user typically needs to push a button or plunger to release the medication.

Common to the above-mentioned types of medical devices, is that the liquid reservoir comprising a drug or medication is arranged in any kind of containment, such as a cartridge. The cartridge typically has a sealing, such as a septum, which serves the purpose to prevent the medication from becoming contaminated. A cannula of the medical device is typically designed to pierce through the septum in order to provide a fluid communication from the liquid reservoir to an injection area of the user.

The septum should be sterile before a needle device is used to pierce through it to provide for a medication to a user. However, common practice in present industries is that while cartridges are being filled with a corresponding drug or medication in a sterile environment, said sterile environment is only provided during the filling process. The outer side of said cartridge and of the septum is typically exposed to a not sterile environment, e.g., for shipping from a supplier to other sites, such as to pharmacists. In said other sites, the cartridge may be mounted to other parts to form a medical injection device. For instance, the cartridge may be mounted to a needle device.

When the septum is exposed to a typical (e.g., not sterile) environment, it is prone to contamination. Sterilization processes for making the septum sterile such as irradiation, autoclaves, gas-sterilization, or the like have the disadvantage that they can damage the cartridge and/or the medication therein. Further, mounting the cartridge on a needle device in a sterile environment is cost-intensive and not practical.

Against this background, improvements for medical devices are needed.

Thus, it is an object of the present disclosure to provide a needle device for a medical device, in particular in a medical injection device, that addresses the aforementioned needs and that overcomes the disadvantages of the present solutions at least partially. Further, it is an object to provide a needle device unit, a medical device, a medical device system and a method for assembling the medical device.

### 3. Summary of the invention

The above-mentioned object is at least partially achieved by the subject-matter of the independent claims. Preferred embodiments are subject of the dependent claims, and other suitable aspects of the present invention are described through the overall disclosure of the present application.

### Needle device

The object is achieved by a needle device for a medical device, in particular for a medical injection device, the needle device comprising: a hub portion defining a longitudinal axis, wherein the hub portion comprises a cannula extending from the hub portion substantially along the longitudinal axis; a tubular portion arranged at an outer periphery of the hub portion; wherein the needle device is releasably attachable to a housing of a medical device.

In this manner, the present disclosure provides an improved needle device compared to known needle devices. In particular, attributable to the needle device being releasably attachable to a housing of a medical device, said needle device can be conveniently attached to said housing and/or removed from the housing whenever this is desired. This may have the advantage that the needle device and a main body of a medical device may be provided separate. The main body may comprise the housing, wherein the housing may comprise the cartridge. This may have various practical advantages, as the outside of the cartridge of the medical device may not necessarily be provided in a sterile manner throughout the time period from filling the cartridge (e.g., with a medicament), and delivering the cartridge to another site for being assembled in the housing of a medical device. Assembling the cartridge with the housing in a sterile manner is typically cost intensive and for practical reasons not performed. The present disclosure allows that the cartridge may not need to be assembled in a sterile manner, as it could be done by a user before assembly. Thereby, the housing with the cartridge may be provided at reduced costs.

Further, the needle device may be easily provided as a separate part, which can be easily sterilized in a simplified manner. That is, because there may not be a critical part such as a cartridge, which could suffer from damage due to a sterilization process such as irradiation, autoclaves, gas-sterilization, or the like. For instance, the needle device could be sterilized in a cost-saving method like irradiation or the like. Preferably, the attachment of the needle device to the housing may be released after performing an injection process by a user.

Overall, the advantage of the present disclosure is that contamination of a liquid reservoir comprising a medication can be mitigated or reduced in a cost-saving and convenient manner. In some cases, such contamination can be fully prevented.

The "tubular portion" may be a separate part or may be integrally formed with another part of the needle device. Preferably, the tubular portion is a separate part. The tubular portion may be a component or structure having a cylindrical or tube-like shape. The tubular portion may define a hollow portion, such as a chamber within its periphery. In one example, the hollow portion defined by the tubular portion may extend through the tubular portion along the longitudinal axis.

The "hub portion" as used in the present disclosure may be an elongated hub portion. In some examples, the longitudinal axis of the hub portion may also be a longitudinal center axis of the hub portion. This may particularly be the case if the hub portion has a substantially cylindrical shape. It is understood that manufacturing tolerances may need to be considered. Thus, even when the hub portion is said to have a cylindrical shape, the shape of the hub portion may slightly deviate from a cylindrical shape. In some examples, a cross section of the hub portion may have a substantially annular shape.

The "cannula" as used in the present disclosure may additionally or alternatively be referred to as a needle or injection needle. The cannula may be hollow, so as to guide a fluid therethrough. Further, the cannula may be attached directly or indirectly to the hub portion. The cannula may be part of a cannula arrangement. For instance, the cannula arrangement may comprise a plurality of cannulas. The plurality of cannulas may be connected to one another, for instance along one direction, in particular along the longitudinal axis of the hub portion. The plurality of cannulas may partially overlap one another along the longitudinal axis, which may serve the purpose to fix the cannula arrangement.

The needle device being "releasably attachable" to a housing may have the advantage that a secure connection is allowed while at the same time a quick release of the needle device and the housing can be ensured.

In one example, the needle device as described in here may comprise a needle safety device. In one example, the needle device as described in here may be a needle safety device.

It is understood that the described advantages may apply also for the following preferred embodiments.

In a preferred embodiment of the needle device of the present disclosure, the tubular portion is releasably attachable to the housing by means of a snap geometry, a screw, a bayonet lock, and / or an adhesive. This facilitates that the needle device can be easily attached and released from the housing. This may simply be performed by the user. It is noted that when an adhesive is used, the adhesive is configured so as to allow for a releasable attachment.

It is noted that various means for attachment of the tubular portion and the housing may be encompassed in the present disclosure. Such attachment means include one or more or all of the following non-exhaustive list: fasteners (such as screws, bolts, nuts, rivets, clamps, staples), releasable adhesives and glues (various types of adhesive substances may bond together when they dry or cure, a double-sided tape comprising adhesive on both sides of a tape), mechanical connections (latches and catches, hooks and loops, zip ties), magnetic attachments, snap-fit connections (interlocking components that snap together), positive locking fits, bayonet locks. As understood by the skilled person, the way in which the two parts are attached to one another may depend on the materials involved and/or the specific intended purpose.

It may be the case that whenever reference is made in the present disclosure to two or more parts being attached, the aforementioned attachment means may be applicable.

In a preferred embodiment of the needle device of the present disclosure, the tubular portion comprises a recess at a proximal end of the tubular portion along the longitudinal axis, preferably a circumferential recess, configured to engage with a corresponding projection, preferably a circumferential projection, at a distal end of a housing of a medical device. The proximal end of the tubular portion may face the medical device when the needle device is attached to the housing. It is noted that a distal end of the tubular portion may be substantially opposite to the proximal end. The distal end of the tubular portion may be closer to an injection area during ordinary use of the medical device.

The "recess" may be an indentation, a hole or the like. The recess may be a macroscopic recess, such that it is configured to accommodate the respective protrusion at least partially. In some examples, the recess may be implemented as a cutout. The recess facilitates a simplified engagement of the needle device and the housing, which can easily be released.

The "projection" of the housing and/or any projection of another part described in here may be a structure that protrudes from the housing or another part, respectively. The projection may be integrally formed with the housing or another part, respectively, or it maybe provided as a separate part. The projection has the advantage of improving attaching of the needle device and the housing. The projection provides the benefit of a secured, and precise fit. This can enhance the stability and reliability of the attachment, mitigating the risk of loosening, misalignment, or disassembly.

In a preferred embodiment of the needle device of the present disclosure, the tubular portion comprises a projection, such as a rib, ridge, or the like, along the longitudinal axis, configured to engage with a corresponding recess along the longitudinal axis of the housing of the medical device. The projection and/or the recess may be implemented in a similar fashion as the respective projection and/or recess described in the foregoing preferred embodiment. This further contributes to an improved attachment, which can be easily released.

In a preferred embodiment of the needle device of the present disclosure, the hub portion comprises an abutting surface configured to axially abut a distal end of a casing of a cartridge of a medical device, when the needle device is attached to the housing of the medical device. This contributes to a relatively smooth alignment of the needle device with the cartridge, in particular with a casing of the cartridge. In one example, the hub portion may not extend further towards the proximal direction, when attached to the housing. Thus, said abutting surface may in one example define a proximal end of the hub portion. The abutting surface may ensure stability, and reliability of a correct positioning of the needle device. Moreover, a substantially equal pressure may be provided on the hub portion, contributing to reduced stresses on the hub portion and the casing of the cartridge. The abutting surface may be substantially flat. In one example, the abutting surface may be shaped substantially in correspondence to the shape of a respective surface of the casing of the cartridge. It is noted that the abutting surface and the surface of the casing of the cartridge being abutted may have the shape of a ring. This may facilitate that the cannula can be guided therethrough.

In a preferred embodiment of the needle device of the present disclosure, the cannula is configured to be moved along the longitudinal axis relative to the hub portion. It is noted that when one or more parts are described in here to "move along the longitudinal axis", this may in some examples mean that the movement is exclusively along the longitudinal axis. However, in other examples, it does not exclude that a movement along an axis not parallel to the longitudinal axis is additionally possible. As understood, in some cases, such a movement along a different axis may be desirable. This may depend on the specific use case of the needle device. The cannula may preferably be permanently attached to the hub portion. Thus, for instance, a replacement of the cannula of the needle device cannot be performed. This may increase safety to the user. That is, because once used, the user may simply exchange the (used) needle device with a new sterile needle device. Any contamination of a cannula can thus be mitigated and/or avoided.

In a preferred embodiment of the needle device of the present disclosure, the needle device is configured such that the cannula does not pierce through a septum of a cartridge of the housing upon attaching the needle device to the housing. This may have the advantage that the cartridge may remain in a sealed condition. For instance, the cartridge may remain in a sealed condition until the user may activate the medical injection device. Such activation may include a movement of the cannula such that it pierces through the septum.

### Needle device unit

The object of the present disclosure is also achieved by a needle device unit, the unit comprising: a needle device as described in the present disclosure, and a containment, wherein the needle device is provided in the containment for storing the needle device in a substantially sealing manner. Since the needle device unit comprises the needle device as described elsewhere herein, it is understood that the features and/or advantages described with reference to the needle device may also apply for the needle device unit and vice versa. Storing in a sealing manner may mean that the needle device is stored in a way that entry of air, moisture, dust, or other external contaminants may be prevented. This may improve longevity of the needle device.

In a preferred embodiment of the needle device unit of the present disclosure, the unit is further comprising: a removable lid for closing the containment. This allows for a simplified closure and opening of the containment, which is appreciated by the user. Further, such a lid may be manufactured in a simplified manner. The removable lid may not require any kind of special equipment or tools to be opened but could easily be grabbed with the hand of a user. Further, removable lids can be more cost-effective than other packaging solutions. The containment and/or the removable lid may be made from recyclable or reusable materials, which is beneficial for the environment.

In a preferred embodiment of the needle device unit of the present disclosure, the unit is configured such that the needle device can be stored in a sterile manner. This further complements the advantages outlined elsewhere. It is understood that the needle device may be sterilized in a cost-effective manner, e.g., by way of irradiation, and then stored in the needle device unit. This makes the overall process of manufacturing and providing a sterile needle device more cost effective.

In a preferred embodiment of the needle device of the present disclosure, the containment is a substantially tubular containment. The tubular containment can provide improved strength and stability, making the containment suitable for containing or supporting the needle device. The tubular containment may be specifically tailored to meet various sizes of needle devices. The tubular shape may have the further advantage of providing an easy access for the user, such that the user may easily take out the needle device thereof.

In a preferred embodiment of the needle device unit of the present disclosure, the containment is a blister. A blister may be understood as a pre-formed plastic mould. Said blister may facilitate a clear visibility of the needle device stored therein. This may be of advantage such that the needle device could be checked and/or monitored easily by a consumer. However, the blister may alternatively be provided with a covering such that the needle device may not be visible. The blister may have the additional advantage of protecting the needle device, for instance during transport thereof and/or storage. In one example, the blister may prevent tampering. The blister may provide a secure and hygienic containment for the needle device.

### Medical device and medical device system

The object of the present disclosure is also achieved by a medical device, in particular a medical injection device, the medical device comprising: a needle device as described in the present disclosure; and a housing accommodating a cartridge comprising a liquid reservoir and a septum for sealing the liquid reservoir; wherein the cartridge is preferably provided in a casing having a distal end in proximity of which the septum is arranged and a proximal end; wherein, when the needle device is not attached to the housing, the septum is preferably accessible for a user; wherein the needle device is preferably configured such that the cannula does not pierce through a septum of a cartridge of the housing upon attaching the needle device to the housing. Since the medical device comprises the needle device as described elsewhere herein, it is understood that the features and/or advantages described with reference to the needle device may also apply for the medical device and vice versa. The septum being accessible for a user may mean that the septum is at least partially exposed to the outside.

In a preferred embodiment, the medical device is an auto-injector or an injection pen.

The object of the present disclosure is also achieved by a medical device system, in particular a medical injection device system, the medical device system comprising: a medical device as described in here; disinfection means configured to allow a user to disinfect the septum before attaching the needle device to the housing of the medical device; wherein the disinfection means is preferably one or more of the following: a disinfection swab, a disinfection rod. Since the medical device system comprises the medical device as described elsewhere herein, it is understood that the features and/or advantages described with reference to the medical device may also apply for the medical device system and vice versa.

The disinfection means facilitates user convenience, as it allows the user to easily make the septum sterile. This is another addition to the benefits described elsewhere herein. Disinfection may be performed for instance by contacting the septum at least partially with at least a part of the disinfection means. The disinfection means may comprise means that facilitate a user to make the septum sterile without contacting the septum. For instance, the disinfection means may comprise using UV light or the like.

### Method

The object of the present disclosure is also achieved by a method for assembling a medical device, in particular a medical injection device, such as a medical device as described in here, the method comprising the following steps:
a. providing a housing of a medical device, the housing accommodating a cartridge comprising a liquid reservoir and a septum for sealing the liquid reservoir;
b. bringing disinfection means in communication with the septum for substantially disinfecting the septum;
c. releasably attaching a sterile needle device, such as a needle device as described in here, to the housing.

The respective advantages described with reference to the needle device, the needle device unit, medical device, and the medical device system may also apply for the method.

In a preferred embodiment, the method is further comprising the following step before step c: providing a needle device unit as described in here; and removing the sterile needle device provided in the needle device unit, for providing the sterile needle device as described in here.

In a preferred embodiment, the method is further comprising removing a lid from a containment of the needle device unit, preferably before step c, and preferably after step b.

### 4. Brief description of the accompanying figures

In the following, the invention will be described in more detail with reference to the following figures:
- **Fig. 1:**: shows a medical device system and parts thereof according to the present invention in a perspective view.
- **Fig. 2:**: shows a needle device unit with a needle device in different steps during removal of the needle device from the needle device unit.
- **Fig. 3:**: shows a needle device and a housing with a cartridge not attached to one another, e.g., before attaching and/or after releasing, in a perspective view.
- **Fig. 4:**: shows Fig. 3 in a cross-sectional view.
- **Fig. 4a:**: shows the needle device and the housing with the cartridge of Fig. 4 when attached.

### 5. Detailed description of the figures

In the following only some possible embodiments of the invention are described in detail. However, the present invention is not limited to these, and a multitude of other embodiments are applicable without departing from the scope of the invention. The presented embodiments can be modified in a number of ways and combined with each other whenever compatible and certain features may be omitted in so far as they appear dispensable. In particular, the disclosed embodiments may be modified by combining certain features of one embodiment with one or more features of another embodiment.

Throughout the present figures and specification, the same reference numerals refer to the same elements. For the sake of clarity and conciseness, certain aspects of components or steps of certain embodiments are presented without undue detail where such detail would be apparent to those skilled in the art in light of the teachings herein and / or where such detail would obfuscate an understanding of more pertinent aspects of the embodiments.

### Definitions

The "proximal" end as used herein is merely used to illustrate one of two ends of a part. The proximal end of a part may be understood best in combination with a distal end of the same part. The terms proximal and distal are not to be understood limiting by any means. The ends could additionally or alternatively be referred to as first and second end or second and first end, respectively.

The distal end of a part may usually be an end that is further away from a center point of the medical device compared to the proximal end of the part. Further, the distal end of a part may usually be an end that is closer to an injection area compared to the proximal end of the part when the medical device is used according to its ordinary use.

Same applies to the terms "proximal direction" and "distal direction". That is, these terms could additionally or alternatively be referred to as first and second direction or second and first direction. The proximal direction may be a direction from a distal end to a proximal end. The distal direction may be a direction from a proximal end to a distal end.

Unless otherwise stated, the term "substantial" or "substantially" as used in the present context may be understood to a great or significant extent or for the most part or essentially. In particular, manufacturing tolerances are included by this term.

### Description of preferred embodiments

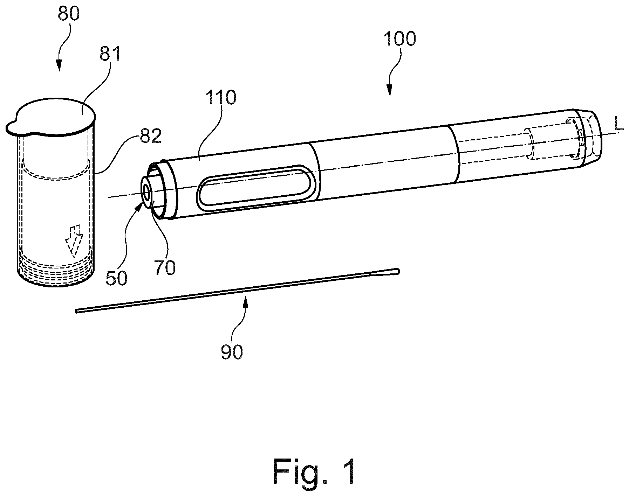

**Fig. 1** shows a medical device system and parts thereof according to the present invention in a perspective view. The medical device system comprises a medical device, in particular a medical injection device 100. In Fig. 1, the medical injection device 100 is not assembled, i.e., a needle device 1 is not attached to a housing 110 of the medical injection device 100. The housing 110 of the medical injection device 100 comprises a cartridge 70 comprising a septum 50. The medical device system further comprises disinfection means 90. Said disinfection means 90 is configured to allow a user to disinfect the septum 50 before attaching the needle device 1 to the housing 110 of the medical device. As can be seen in Fig. 1, the disinfection means 90 may be a disinfection swab and/or a disinfection rod.

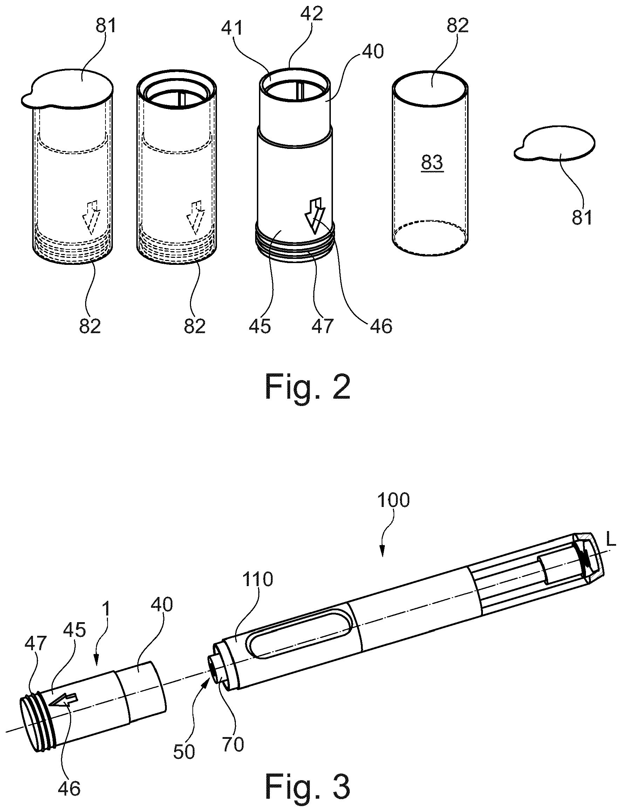

**Fig. 2** shows a needle device unit 80 with a needle device 1 in different steps of removing the needle device 1 from the needle device unit 80. The unit 80 comprises the needle device 1 as can be seen in the left two images of Fig. 2. The unit 80 also comprises a containment 82. In the right three images of Fig. 2, the needle device 1 is not provided in the containment 82 for storing the needle device 1 anymore. In the left image of Fig. 2, the needle device 1 may be stored in the containment 82 in a substantially sealing manner. Further, the unit 80 comprises a removable lid 81 for closing the containment 82. The lid 81 may in some examples be closable again. However, preferably, the lid 81 is a disposable lid 81, which ensures that once the unit 80 is opened, it cannot be closed anymore such that contamination and/or incorrect use can be prevented. The containment 82 may define a cavity 83, e.g., an empty space, in which the needle device 1 may be stored. As can be gathered from Fig. 2, the containment 82 may be a substantially tubular containment. As can be gathered from the second image from the right-hand side of Fig. 2, the containment 82 may be a blister.
**Fig. 3** shows a needle device 1 and a housing 110 of a medical device 100 with a cartridge 70, wherein the needle device 1 is not attached to the housing 110. For instance, Fig. 3 shows a state before attaching and/or after releasing said attachment. Fig. 3 and Fig. 1 also show a longitudinal axis L of the housing 110 of the medical injection device 100.

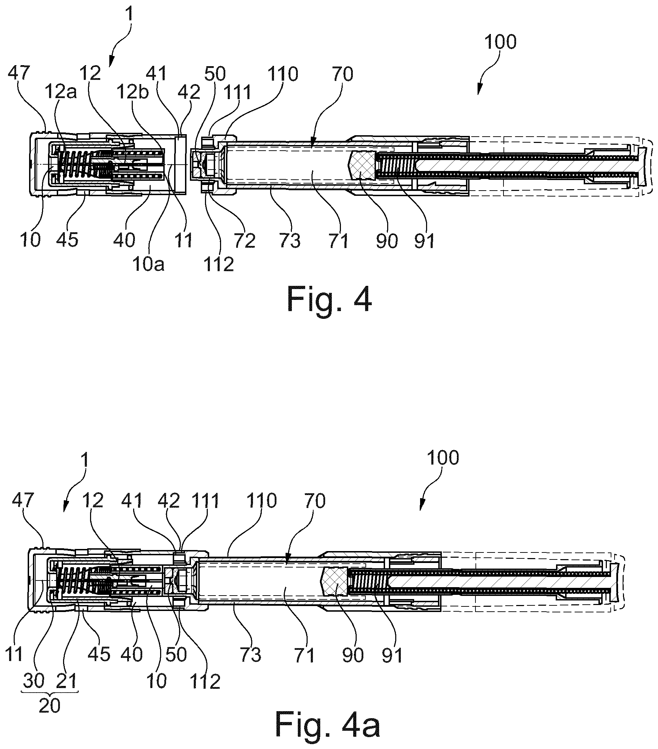

**Fig. 4** shows Fig. 3 in a cross-sectional view. **Fig. 4a** shows the needle device 1 and the housing 110 of the medical device 100 of Fig. 4 when attached.

As described elsewhere herein, the medical device may be in particular a medical injection device 100. The medical device comprises the needle device 1 and the housing 110 accommodating the cartridge 70 comprising a liquid reservoir 71 and a septum 50 for sealing the liquid reservoir 71. The cartridge 70 may be provided in a casing 73 having a distal end in proximity of which the septum 50 is arranged and a proximal end. When the needle device 1 is not attached to the housing 110 (Fig. 3 and 4), the septum 50 may be accessible for a user. In this manner, the user may easily use the disinfection means 90 (Fig. 1) so as to disinfect the septum 50. Further, as can be gathered from Fig. 4a, the needle device 1 may be configured such that the cannula 12 does not pierce through the septum 50 of the cartridge 70 of the housing 110 upon attaching the needle device 1 to the housing 110. It is noted that the cannula 12 may remain in such a state, i.e., not piercing through the septum 50, until the user may activate the medical device 100. As understood, not piercing through the septum 50 may mean that a fluid communication from the liquid reservoir 71 of the cartridge 70 to an injection area of the user may not be provided. This may improve sealing and/or prevent contamination.

The needle device 1 comprises a hub portion 10 defining a longitudinal axis 11, wherein the hub portion 10 comprises a cannula 12 extending from the hub portion 10 substantially along the longitudinal axis 11. The needle device 1 further comprises a tubular portion 40 arranged at an outer periphery of the hub portion 10. The needle device 1 is releasably attachable to the housing 110 of the medical injection device 100.

In the prior art, the sterilization methods usually employed have the following disadvantages: (1) irradiation can usually not be used for glass-based cartridges, since irradiation may induce color change in the glass and may also have an impact on the medication comprised in the liquid reservoir of the cartridge. (2) Autoclaves may run at about at least 120°C for longer periods of time, such as about 30 min, which could lead to damage of the medication. (3) Gas-sterilization, for instance using Ethylene Oxide (EtO) is also run at higher temperatures, e.g., about at least 30°C for many hours, which may adversely affect the medication. (4) Gas-sterilization methods like NO2 are not quite common in this technical field and cost-intensive.

One particular contribution of the present disclosure is that the above-mentioned sterilization methods can be easily employed to the needle device 1, preferably before being stored in the containment 81 in a substantially sterile manner as part of the needle device unit 80. Such sterilization may be performed without jeopardizing any adverse effects to the medication. The housing 110 with the cartridge 70 may be provided as separate parts. The septum 50 may be disinfected by the user easily and then the needle device 1 may be releasable attached to the housing 110 of the medical injection device 100.

The tubular portion 40 may be releasably attachable to the housing 110 by means of a snap geometry, a screw, and / or an adhesive. Further suitable means for releasably attaching the needle device to the housing as used in the present disclosure may include one or more of the followings: buckles, quick-release pins, snap fasteners, latches, cable ties, spring clips, quick-connect couplings, push-to-connect fittings, snap hooks, carabiners, zip ties, magnetic clasps, Velcro, toggle clamps. Depending on the specific needle device, the releasable attachment may vary.

As can be seen from **Fig. 4** and **Fig. 4a**, the tubular portion 40 may have an elongated shape. The term "elongated" means that there may be a dimension along one axis, which may be larger than one and preferably than both dimensions along the remaining axes, the remaining axes being substantially perpendicular to said one axis. It is understood that when dimensions are described herein, manufacturing tolerances usually have to be taken into consideration. Thus, the dimensions described herein may vary slightly.

The tubular portion 40 may comprise a recess 41 at a proximal end of the tubular portion 40 along the longitudinal axis 11, preferably a circumferential recess 41. This recess 41 may be configured to engage with a corresponding projection 112, preferably a circumferential projection 112 of the housing 110. The projection 112 may be arranged at a distal end of the housing 110 as shown in Fig. 4 and Fig. 4a.

Alternatively, or additionally, the tubular portion 40 may comprise a projection 42, such as a rib, ridge, or the like, along the longitudinal axis 11, configured to engage with a corresponding recess 111 of the housing 110 along the longitudinal axis 11.

As can be seen from **Fig. 4a**, the hub portion 10 comprises an abutting surface 10a configured to axially abut a distal end of the casing 73 of the cartridge 70 of the medical injection device 100, when the needle device 1 is attached to the housing 110.

As described elsewhere herein, the liquid reservoir 71 may be within the cartridge 70. The cartridge 70 may have the shape of a barrel or the like. Further, as described elsewhere herein, the liquid reservoir 71 may be housed within a casing 73 of the cartridge 70. Said casing 73 may in some examples be made of glass or plastic. The casing 73 may be substantially cylindrically. The liquid reservoir 71 may comprise a medication or a component thereof. The liquid reservoir 71 may be located in proximity to the septum 50. Further, the cartridge 70 may comprises a clamp 72 (Fig. 4) arranged at least partially around the septum 50. The clamp 72 may substantially hold the septum 50 in its place. A fluid communication from the liquid reservoir 71 to an injection area of the user may be provided as the cannula 12 of the needle device 1 pierces through the septum 50. Thereby, a medication can be delivered to said injection area of the user. It is noted that the cannula 12 may be any kind of piercing means, such as a needle, or the like. Moreover, the cannula 12 may be configured to be moved along the longitudinal axis 11 relative to the hub portion 10.

The septum 50 may prevent air, contaminants, and moisture from entering the liquid reservoir 71 and affecting the stability and efficacy of the medication. Typically, when the medical injection device 100 is activated, the cannula 12 pierces or punctures the septum 50 to access the medication comprised in the liquid reservoir 71 of the cartridge 70. This piercing or puncturing facilitates the medication to be drawn out of the liquid reservoir 71. The septum 50 may ensure that the medication remains sterile until the moment of administration.

As can be seen from **Fig. 3****,** **Fig. 4, and Fig. 4a**, the tubular portion 40 may be a tubular casing. The tubular portion 40 may be an outermost part of the needle device **1.** In some examples, the tubular portion 40 may be a first tubular portion 40, or a first tubular casing 40 (in the following reference is made to the portion 40 only but it is understood to apply to both, i.e., the portion 40 and the casing 40) and the needle device 1 may comprise a second tubular portion 45 or a second tubular casing 45 (in the following reference is made to the portion 45 only but it is understood to apply to both, i.e., the portion 45 and the casing 45). Said second tubular portion 45 may at least partially accommodate the first tubular portion 40 with an inner periphery of the tubular portion 45. In other words, the first tubular portion 40 may be received at least partially within the second tubular portion 45. The first tubular portion 40 and the second tubular portion 45 may be designed such that they could be easily held by one or more hands of a user.

As can be seen from **Fig. 2, Fig. 3****,** **Fig. 4, and Fig. 4a**, the second tubular portion 45 may comprise an indication 46. Such indication may provide the user with a haptic feedback. This may aid in positioning the needle device 1 in a correct orientation without the need to look at the needle device **1.** In a preferred embodiment, the indication 46 may be an arrow. Further the second tubular portion 45 may comprise a structured area. This may also provide the user with a haptic feedback. Moreover, this may also aid in positioning the needle device 1 in a correct orientation without the need to look at the needle device **1.** The structured area 47 may comprise circumferential ribs. In a preferred embodiment, three circumferential ribs may be comprised by the structured area 47.

In one example, the medical injection device 100 referred to herein may be different compared to insulin pens, as the cannula 12 is not removed from the needle device **1.** The cannula of insulin pens typically needs to be removed after each time the pen has been used for an injection. This is not necessary for the needle device 1 in the present disclosure. For instance, the cannula 12 may be permanently attached to the needle device 1, such as to the hub portion 10 of the needle device **1.**

In one example, the medical injection device 100 referred to herein may be different compared to insulin pens, in that the order in which an injection is performed is different. The needle device 1 of the present disclosure may be used to penetrate an injection area of the user first, before releasably attaching the needle device 1 to the housing 110 of the medical injection device 100. This may be beneficial, as the cartridge 70 of the medical injection device 100 may be pre-pressurized. Said pre-pressurization may be established by means of a biasing element 91 pushing against a plunger 90 in proximity to the liquid reservoir 71 of the cartridge 70 as shown in Fig. 4 and Fig. 4a. Medical injection devices 100 where such a pressure is provided may be referred to as pre-pressurized medical injection devices 100. When employing insulin pens, the user penetrates the septum first, before he or she sets a dose and, subsequently, he or she penetrates the injection area.

As described elsewhere herein, the cannula 12 may be attached to the hub portion 10, Any suitable attachment means are encompassed in the present disclosure. Such attaching and variations thereof includes the joining of the cannula 12 and the hub portion 10 directly or indirectly to one another. The joining may be stationary (e.g., permanent, or fixed) or moveable (e.g., removable, or releasable). The joining may be achieved with the two parts (e.g., cannula 12 and hub portion 10) coupled directly to each other, with the two parts coupled with each other using a separate intervening part and / or any additional intermediate parts coupled with one another, or with the two parts coupled with each other using an intervening part that is integrally formed as a single unitary body with one of the two parts. The attachment may comprise any means of mechanical attachment. Further, the attachment may include a form fit connection of the two parts. This may create a tight and secure fit without the need for additional fasteners or adhesives. Thereby, the attachment may rely on the geometry of the cannula 12 and the hub portion 10 to create a stable and reliable attachment. However, usage of additional means for attachment is not precluded.

In the following, some general features of the needle device 1 of the present disclosure are explained in greater detail, in particular when the needle device 1 is a needle safety device **1.**

The device 1 may comprise a tubular arrangement 20 comprising a first tubular element 21 and a second tubular element 30 (as indicated in Fig. 4a). The first tubular element 21 may be movably arranged on the hub portion 10 to move along the longitudinal axis 11. The first tubular element 21 may comprise a guide track which slidably engages with a guide pin of the hub portion 10. The guide track may be configured such that the first tubular element 21 rotates relative to the hub portion 10 when the guide pin moves along the guide track, wherein the guide track may comprise a final location configured to retain the guide pin. The second tubular element 30 may be arranged on the first tubular element 21 such that the second tubular element 30 is movable along the longitudinal axis 11 together with the first tubular element 21. The first tubular element 21 may be at least partially enclosed by the second tubular element 30. The second tubular element 30 may comprise a contact surface 31 for establishing pressure contact with an injection area of the user.

It is noted that any one or more of the embodiments described herein and / or examples may be combined with further aspects as described herein and details of the embodiments and / or examples may also be omitted, as will be understood by the skilled person. The scope of protection is determined by the claims and is not limited by the embodiments and / or examples disclosed in the above figures.

### List of reference signs

- 1: needle device
- 10: hub portion
- 10a: abutting surface along longitudinal axis
- 11: longitudinal axis
- 12: cannula
- 12a: distal end of the cannula
- 12b: proximal end of the cannula

- 20: tubular arrangement
- 21: first tubular element

- 30: second tubular element

- 40: (first) tubular portion
- 41: (circumferential) recess of tubular portion
- 42: (circumferential) projection of tubular portion
- 45: second tubular portion
- 46: indication of the second tubular portion
- 47: structured area of the second tubular portion

- 50: septum

- 70: cartridge
- 71: liquid reservoir
- 72: clamp
- 73: casing of cartridge

- 80: needle device unit
- 81: lid
- 82: containment
- 83: cavity of the containment

- 90: plunger
- 91: biasing element for the plunger

- 100: medical injection device
- 110: housing
- 111: recess of the housing of the medical injection device
- 112: projection of the housing of the medical injection device

- L: longitudinal axis of the housing of the medical injection device

## Claims

1. A needle device for a medical device, in particular for a medical injection device, the needle device (1) comprising:
a hub portion (10) defining a longitudinal axis (11), wherein the hub portion (10) comprises a cannula (12) extending from the hub portion (10) substantially along the longitudinal axis (11);
a tubular portion (40) arranged at an outer periphery of the hub portion (10);
wherein the needle device (1) is releasably attachable to a housing (110) of a medical device.

2. The needle device (1) according to the preceding claim, wherein the tubular portion (40) is releasably attachable to the housing (110) by means of a snap geometry, a screw, a bayonet lock and / or an adhesive.

3. The needle device (1) according to any one of the preceding claims, wherein the tubular portion (40) comprises a recess (41) at a proximal end of the tubular portion along the longitudinal axis, preferably a circumferential recess (41), configured to engage with a corresponding projection (112), preferably a circumferential projection (112), at a distal end of a housing (110) of a medical device.

4. The needle device (1) according to any one of the preceding claims, wherein the tubular portion (40) comprises a projection (42), such as a rib, ridge, or the like, along the longitudinal axis, configured to engage with a corresponding recess (111) along the longitudinal axis of the housing (110) of the medical device.

5. The needle device (1) according to any one of the preceding claims, wherein the hub portion (10) comprises an abutting surface (10a) configured to axially abut a distal end of a casing of a cartridge (70) of a medical device, when the needle device (1) is attached to the housing (110) of the medical device.

6. The needle device (1) according to any one of the preceding claims, wherein the cannula (12) is configured to be moved along the longitudinal axis (11) relative to the hub portion (10),
wherein the needle device (1) is preferably configured such that the cannula (12) does not pierce through a septum (50) of a cartridge (70) of the housing (110) upon attaching the needle device (1) to the housing (110).

7. A needle device unit (80), the unit comprising:
a needle device (1) according to any one of the preceding claims, and
a containment (82), wherein the needle device (1) is provided in the containment (82) for storing the needle device (1) in a substantially sealing manner.

8. The needle device unit (80) according to the preceding claim, the unit (80) further comprising:
a removable lid (81) for closing the containment (82).

9. The needle device unit (80) according to any one of claims 7 or 8, wherein the unit (80) is configured such that the needle device (1) can be stored in a sterile manner.

10. The needle device unit (80) according to any one of claims 7 to 9, wherein the containment (82) is a substantially tubular containment (82).

11. The needle device unit (80) according to any one of claims 7 to 10, wherein the containment (82) is a blister.

12. A medical device, in particular a medical injection device (100), the medical device comprising:
a needle device (1) according to any one of the preceding claims; and
a housing (110) accommodating a cartridge (70) comprising a liquid reservoir and a septum (50) for sealing the liquid reservoir;
wherein the cartridge (70) is preferably provided in a casing having a distal end in proximity of which the septum (50) is arranged and a proximal end;
wherein, when the needle device (1) is not attached to the housing (110), the septum (50) is preferably accessible for a user;
wherein the needle device (1) is preferably configured such that the cannula (12) does not pierce through a septum (50) of a cartridge (70) of the housing (110) upon attaching the needle device (1) to the housing (110).

13. A medical device system, in particular a medical injection device system (100), the medical device system comprising:
a medical device according to the preceding claim;
disinfection means configured to allow a user to disinfect the septum (50) before attaching the needle device (1) to the housing (110) of the medical device;
wherein the disinfection means is preferably one or more of the following: a disinfection swab, a disinfection rod.

14. A method for assembling a medical device, in particular a medical injection device (100), such as a medical device according claim 12, the method comprising the following steps:
a. providing a housing (110) of a medical device, the housing (110) accommodating a cartridge (70) comprising a liquid reservoir and a septum (50) for sealing the liquid reservoir;
b. bringing disinfection means in communication with the septum (50) for substantially disinfecting the septum (50);
c. releasably attaching a sterile needle device (1), such as a needle device (1) according to any one of claims 1 to 6, to the housing (110).

15. The method according to the preceding claim, the method comprising before step c:
providing a needle device unit (80) according to claims 7 to 11; and
removing the sterile needle device (1) provided in the needle device unit (80), for providing the sterile needle device (1) according to any one of claims 1 to 6.
